# EUROPÄISCHE PATENTSCHRIFT

(11) **EP 0 449 089 B1**
(45) Veröffentlichungstag und Bekanntmachung des Hinweises auf die Patenterteilung: **12.01.1994**
(21) Anmeldenummer: 91104288.5
(22) Anmeldetag: 20.03.1991
(51) Int. Cl.: C07C 211/36, C07C 209/00, C07C 209/26, C07C 209/48

(54) **Verfahren zur Herstellung von 3-Aminomethyl-3,5,5,-trimethyl-cyclohexylamin**
Process for preparing 3-aminomethyl-3,5,5-trimethyl-cyclohexylamine
Procédé pour la préparation de l'aminométhyl-3 triméthyl-3,5,5 cyclohexylamine

(30) Priorität: 30.03.1990 DE 4010227
(43) Veröffentlichungstag der Anmeldung: 02.10.1991
(73) Patentinhaber: BASF Aktiengesellschaft, 67063 Ludwigshafen (DE)
(72) Erfinder: Merger, Franz, Dr., W-6710 Frankenthal (DE); Priester, Claus-Ulrich, Dr., W-6700 Ludwigshafen (DE); Witzel, Tom, Dr., W-6700 Ludwigshafen (DE); Koppenhoefer, Gerhard, Dr., W-6725 Roemerberg (DE); Harder, Wolfgang, Dr., W-6940 Weinheim (DE)

(56) Entgegenhaltungen:
- EP-A- 0 042 119
- EP-A- 0 394 968
- DE-A- 3 011 656
- GB-A- 0 972 010
- CHEMICAL ABSTRACTS, Band 109, Nr. 11, 12. September 1988, Columbus, Ohio, USA; Y. HIRAKO: "3-Aminomethyl-3,5,5-Trimethylcyclohexylamine", Seite 647, Zusammenfassung Nr. 92 344k; & JP-A-62 123 154

## Beschreibung

Die vorliegende Erfindung betrifft ein neues Verfahren zur Herstellung von 3-Aminomethyl-3,5,5-trimethyl-cyclohexylamin aus 3-Cyano-3,5,5-trimethyl-cyclohexanon.

Aus der EP-A-42 119 ist ein Verfahren zur Herstellung primärer Mono- und Diamine aus Oxoverbindungen, die gegebenenfalls auch weitere zur Reduktion befähigte Gruppen enthalten können, mit Ammoniak und Wasserstoff in Gegenwart bekannter Hydrierkatalysatoren bekannt, bei dem vor der Reaktion mit Ammoniak und Wasserstoff in Gegenwart von Hydrierkatalysatoren die Oxo-Verbindungen bei Temperaturen von 10 bis 200°C und Drücken von 1 bis 300 bar einer Vorreaktion mit Ammoniak in Gegenwart von anorganischen und organischen Ionenaustauschern in der Ammoniumform als Iminbildungskatalysatoren unterwirft. Nach diesem Verfahren wurde 3-Cyano-3,5,5-trimethyl-cyclohexanon (Isophoronnitril) durch Umsetzung an Ionenaustauschern und durch konventionelle Hydrierung an konventionellen Cobaltkatalysatoren in Ausbeuten bis 95 % in 3-Aminomethyl-3,5,5-trimethyl-cyclohexylamin (Isophorondiamine) überführt.

Dieses Verfahren hat den Nachteil, daß zur Erzielung kurzer Verweilzeiten in der Iminierungsstufe Ionenaustauscher benötigt werden. Die hierbei zur Erzielung kurzer Verweilzeiten in der Isomerisierungsstufe benötigten Ionenaustauscher (organische oder anoganische, wie Zeolithe) haben neben hohen Kosten den Nachteil mangelnder mechanischer bzw. thermischer Stabilität.

Der vorliegenden Erfindung lag daher die Aufgabe zugrunde, ein Verfahren zur Herstellung von 3-Aminomethyl-3,5,5-trimethyl-cyclohexylamin aus 3-Cyano-3,5,5-trimethyl-cyclohexanon zu finden, das dem zuvor genannten Nachteil abhilft.

Demgemäß wurde ein neues und verbessertes Verfahren zur Herstellung von 3-Aminomethyl-3,5,5-trimethyl-cyclohexylamin aus 3-Cyano-3,5,5-tri-methyl-cyclohexanon gefunden, welches dadurch gekennzeichnet ist, daß man in zwei räumlich voneinander getrennten Reaktionsräumen
a) das 3-Cyano-3,5,5-trimethyl-cyclohexanon in einem ersten Reaktionsraum mit überschüssigem Ammoniak an aciden Metalloxidkatalysatoren bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an cobalt-, nickel-, ruthenium- und/oder anderen edelmetallhaltigen Katalysatoren gegebenenfalls mit basischen Komponenten oder auf neutralen oder basischen Trägern bei Temperaturen von 60 bis 150°C und Drücken von 50 bis 300 bar hydriert.

Das erfindungsgemäße Verfahren läßt sich wie folgt in zwei räumlich voneinander getrennten Reaktionsräumen durchführen:

a) In einer ersten Verfahrensstufe setzt man 3-Cyano-3,5,5-trimethyl-cyclohexanon mit überschüssigem Ammoniak bei Temperaturen von 20 bis 150°C, vorzugsweise von 30 bis 130°C, besonders bevorzugt von 50 bis 100°C und Drücken von 15 bis 500 bar, vorzugsweise von 100 bis 350 bar zu den 3-Cyano-3,5,5-trimethyl-cyclohexyliminen um.

Als acide Metalloxidkatalysatoren eignen sich Aluminiumoxid, Siliciumdioxid, Titandioxid, Zirkondioxid, Bevorzugt verwendet man Aluminiumoxid, Titandioxid, Zirkondioxid, insbesondere Aluminiumoxid und Titandioxid. Die Acidität der Katalysatoren kann gegebenenfalls durch Dotierung mit Halogeniden erhöht werden. So finden z.B. auch halogendotierte Katalysatoren, wie Chlorid auf Aluminiumoxid oder Chlorid auf Titandioxid, Verwendung.

Bei der Iminierung hält man eine Katalysatorbelastung von 0,01 bis 10, vorzugsweise von 0,05 bis 7, besonders bevorzugt von 0,1 bis 5 kg 3-Cyano-3,5,5-trimethyl-cyclohexanon pro kg Katalysator und Stunde ein. Pro Mol 3-Cyano-3,5,5-trimethyl-cyclohexanon setzt man bei der Iminierung zweckmäßig, aber nicht zwingend 5 bis 500 Mol NH₃, bevorzugt 10 bis 400 Mol, besonders bevorzugt 20 bis 300 Mol ein. Die Iminierung der 3-Cyano-3,5,5-trimethyl-cyclohexanon kann auch in Anwesenheit eines Lösungsmittels, wie z.B. Alkanolen oder Tetrahydrofuran durchgeführt werden.

Die Iminierung wird bevorzugt kontinuierlich durchgeführt, z.B. in Druckbehältern oder Druckbehälterkaskaden. Nach einer besonders bevorzugten Ausführungsform werden die 3-Cyano-3,5,5-trimethyl-cyclohexanon und NH₃ durch einen Rohrreaktor geleitet, in dem der Iminierungskatalysator in Form eines festen Bettes angeordnet ist.

Die Gesamtverweilzeit in Stufe 1 ergibt sich aus der Katalysatorbelastung und der Menge an eingesetztem Ammoniak. Sie liegt zweckmäßig im Bereich von 0,5 bis 120 Minuten, bevorzugt von 1 bis 40, besonders bevorzugt von 1,5 bis 20 Minuten.

b) Das so erhaltene Produkt wird in einer zweiten Verfahrensstufe mit 3 bis 10.000 Moläquivalenten Wasserstoff, bevorzugt 4,5 bis 30, gegebenenfalls nach Zufuhr von weiterem Ammoniak, einer katalytischen Hydrierung zugeführt.

Bei der aminierenden Hydrierung hält man eine Temperatur von 60 bis 150°C, bevorzugt von 70 bis 140°C, besonders bevorzugt von 80 bis 130°C und einen Druck von 50 bis 500 bar, bevorzugt von 100 bis 350 bar, besonders bevorzugt von 150 bis 300 bar ein.

Die Katalysatorbelastungen liegen zweckmäßig im Bereich von 0,01 bis 5 kg/[kg·h], bevorzugt bei 0,02 bis 2,5, besonders bevorzugt bei 0,05 bis 2 kg/[kg·h].

Die Hydrierung wird vorzugsweise in flüssigem Ammoniak durchgeführt. Pro Mol 3-Cyano-3,5,5-trimethyl-cyclohexylimin setzt man 5 bis 500 Mol NH₃ ein, bevorzugt 10 bis 400 Mol, besonders bevorzugt 20 bis 300 Mol. Zweckmäßigerweise wählt man das NH₃-Angebot, das bei der vorgelagerten Herstellung der 3-Cyano-3,5,5-trimethyl-cyclohexylimin aus den entsprechenden 3-Cyano-3,5,5-trimethyl-cyclohexanon eingestellt wurde. Der NH₃-Anteil kann jedoch auch vor der Hydrierung durch Zugabe von zusätzlichem NH₃ auf den gewünschten Wert erhöht werden.

Die aminierende Hydrierung von 3-Cyano-3,5,5-trimethyl-cyclohexyimin führt man bevorzugt kontinuierlich z.B. in druckfesten Rührbehältern oder in einer Rührbehälterkaskade durch. In einer besonders bevorzugten Ausführungsform werden Rohrreaktoren eingesetzt, in denen das Produktgemisch aus der Iminierung das 3-Cyano-3,5,5-trimethyl-cyclohexanon in Sumpf- oder Rieselfahrweise über ein fest angeordnetes Katalysatorbett geleitet wird.

Die Verfahrensstufen a und b können ebenfalls in einem Reaktor durchgeführt werden, in dem Iminierungskatalysatoren und Hydrierkatalysatoren in zwei getrennten Schichten angeordnet sind. In diesem Fall führt man die Iminierung zweckmäßigerweise in Gegenwart von Wasserstoff durch.

Bei kontinuierlicher Fahrweise im Rohrreaktor ohne Rückführung ergibt sich die Gesamtverweilzeit aus der Katalysatorbelastung und der Menge an eingesetztem Ammoniak. Sie liegt im Bereich von 0,5 bis 120 Minuten, bevorzugt von 1 bis 40, besonders bevorzugt bei 1,5 bis 20 Minuten.

Nach der Hydrierung wird überschüssiges Ammoniak gegebenenfalls unter Druck abgetrennt. Das so erhaltene 3-Aminomethyl-3,5,5-trimethyl-cyclohexylamin läßt sich durch fraktionierende Destillation isolieren.

Bei der Hydrierung können prinzipiell alle gängigen Hydrierkatalysatoren eingesetzt werden, die Nickel, Cobalt, Eisen, Kupfer, Ruthenium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/Aluminiumoxide in Frage, bevorzugt werden Hydrierkatalysatoren mit basischen Komponenten, wie Oxide und Hydroxide von Alkali- und Erdalkalimetallen. Besonders bevorzugt sind daher basische Träger, wie z.B. β-Aluminiumoxid oder Mangesiumoxid/Aluminiumoxide. Besonders bevorzugt ist Magnesiumoxid/Aluminiumoxid mit einem Magnesiumoxidanteil von 5 bis 40 %. Dabei kann der Magnesiumoxid und Aluminiumoxide enthaltende Träger amorph sein oder als Spinell vorliegen.

Die basische Komponente kann ggf. auch während des Hydrierprozesses zugeführt werden, z.B. als Lösung von Alkali- oder Erdalkalihydroxiden in Wasser.

Besonders bevorzugt verwendet man in der Hydrierung Cobalt oder Ruthenium mit basischer Komponente. Diese Katalysatoren erhält man technisch in an sich bekannter Weise. So gewinnt man z.B. Ruthenium auf basischem Träger durch Auftragen von wäßrigen Rutheniumsalz-Lösungen wie Rutheniumchlorid und Rutheniumnitrat auf den entsprechenden Träger. Die Ruthenium-Konzentration auf dem Träger beträgt 0,1 bis 10 %, bevorzugt 0,5 bis 5 %, besonders bevorzugt 1 bis 4 %. Nach Trocknung und gegebenenfalls nach Calcinierung bei Temperaturen von 120 bis 500°C, bevorzugt bei 200 bis 400°C, erfolgt die Aktivierung der Ruthenium-Katalysatoren im Wasserstoffstrom bei Temperaturen von 180 bis 250°C, bevorzugt bei 190 bis 230°C und Drücken von 1 bis 500 bar, bevorzugt von 20 bis 300 bar innerhalb von 1 bis 20 Stunden, bevorzugt 2 bis 10 Stunden.

Die Ruthenium-Katalysatoren können gegebenenfalls noch weitere Metalle enthalten, wie Palladium oder Eisen. Der Eisengehalt liegt dabei im allgemeinen im Bereich von 0,5 bis 5 %, der Palladiumgehalt im Bereich von 0,1 bis 5 %.

Die Ruthenium-Katalysatoren zeichnen sich dadurch aus, daß mit diesen besonders hohe Katalysatorbelastungen realisiert und somit besonders hohe Raum-Zeit-Ausbeuten erzielt werden können.

Die basischen Cobalt-Katalysatoren enthalten mindestens eine basische Komponente wie Li₂O, Na₂O, K₂O, MgO, CaO, SrO oder BaO. Bevorzugt enthalten diese Katalysatoren daneben mindestens eines der Elemente Eisen, Nickel, Mangan, Chrom, Molybdän, Wolfram oder Phosphor. Von besonderem Interesse sind solche Kontakte, die neben Cobalt und einer basischen Komponente mindestens eines der Elemente Eisen, Nickel oder Mangan enthalten. Dabei können die Metalle in metallischer Form oder in Form ihrer Oxide eingesetzt werden. Phosphor liegt praktisch in Form von Phosphorsäure vor.

3-Aminomethyl-3,5,5-trimethyl-cyclohexylamin (Isophorondiamin) ist ein wichtiges Zwischenprodukt für Diisocyanate und Polyamide.

### Beispiele

### Beispiel 1

Ein vertikaler Rohrreaktor (Durchmesser: 16 mm, Füllhöhe: 50 cm, ölbeheizter Doppelmantel) wurde mit 90,1 g (87 ml) eines Katalysators mit 3 % Ruthenium auf β-Aluminiumoxid in Form von 1,2-mm-Strängen gefüllt (Katalysatorherstellung durch Porentränkung von β-Aluminiumoxid mit wäßriger Rutheniumnitrat-Lösung und Trocknen bei 120°C). Zur Reduktion des Katalysators wurde bei 100 bar unter gleichzeitigem Durchleiten von 150 Nl/h Wasserstoff die Temperatur innerhalb 7 h schrittweise von 100 auf 220°C erhöht und dann 9 h bei 220°C gehalten.

Durch einen vor den Hydrierreaktor geschalteten Rohrreaktor (Durchmesser: 16 mm, Füllhöhe: 50 cm, ölbeheizter Doppelmantel) der mit 63,5 g (100 ml) Titandioxid (Anatas) in Form von 1,5 mm-Strängen gefüllt war, werden bei einem Druck von 250 bar und einer Temperatur von 80°C stündlich von unten nach oben 80,4 g einer 50 %igen Lösung von Isophoronnitril in Tetrahydrofuran (Reinheit des IPN 99,0 %) und 303,0 g flüssiges Ammoniak gepumpt. Anschließend werden stündlich 60 Nl/h (2,7 Mol) Wasserstoff zugefahren und der Austrag aus dem vorgeschalteten Iminierungsreaktor bei einem Druck von 250 bar und einer Temperatur von 120°C von unten nach oben durch den Hydrierreaktor gefahren. Nach Entspannen auf Normaldruck wird Ammoniak abdestilliert. Der Hydrieraustrag enthält laut GC 95,2 % Isophorondiamin neben 0,8 % 1,3,3-Trimethyl-6-azabicyclo[3.2.1]octan, entsprechend einer Diamin-Ausbeute von 96,2 %.

### Beispiel 2

Ein vertikaler Rohrreaktor (Durchmesser: 16 mm, Füllhöhe: 50 cm, ölbeheizter Doppelmantel) wurde mit 183,1 g (100 ml) eines Cobalt-Vollkontaktes (CoO mit 5 96 Mn₂O₃ und 3 % P₂O₅) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 70,0 g (100 ml) γ-Aluminimumoxid in Form von 1,5 mm-Strängen gefüllt war, werden bei einem Druck von 250 bar und einer Temperatur von 80°C stündlich von unten nach oben 25,4 g einer 50 %igen Lösung von Isophoronnitril in Tetrahydrofuran (Reinheit des IPN 99,0 %) und 303,0 g flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wird der Ammoniak abdestilliert. der Hydrieraustrag enthält neben THF laut gaschromatographischer Analyse 94,1 % Isophorondiamin und 1,4 % 1,3,3-Trimethyl-6-azabicyclo[3.2.1]octan.

### Beispiel 3

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes (CoO mit 5 % Mn₂O₃ und 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 70,0 g (100 ml) γ-Aluminimumoxid in Form von 1,5 mm-Strängen gefüllt war, werden bei einem Druck von 250 bar und einer Temperatur von 80°C stündlich von unten nach oben 40,4 g einer 50 %igen Lösung von Isophoronnitril in Tetrahydrofuran (Reinheit des IPN 99,0 %) und 303,0 g flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wird der Ammoniak abdestilliert. der Hydrieraustrag enthält neben THF laut gaschromatographischer Analyse 96,0 % Isophorondiamin und 0,6 % 1,3,3-Trimethyl-6-azabicyclo[3.2.1]octan. Der Austrag aus 121 h wird gesammelt und durch fraktionierende Destillation aufgetrennt: man erhält 2371 g Isophorondiamin, entsprechend einer Ausbeute von 95, 1 %.

### Beispiel 4

Ein vertikaler Rohrreaktor (Durchmesser: 16 mm, Füllhöhe: 50 cm, ölbeheizter Doppelmantel) wurde mit 176,7 g (100 ml) eines basischen Cobalt-Vollkontaktes (CoO mit 5 % Mn₂O₃ und 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 50 cm, ölbeheizter Doppelmantel), der mit 70,0 g (100 ml) γ-Aluminimumoxid in Form von 1,5 mm-Strängen gefüllt war, werden bei einem Druck von 250 bar und einer Temperatur von 80°C stündlich von unten nach oben 13,8 g geschmolzenes Isophoronnitril (Reinheit 99,0 %) und 303,0 g flüssiges Ammoniak gepumpt. Anschließend werden stündlich 60 Nl/h (2,7 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wird der Ammoniak abdestilliert. Der Hydrieraustrag enthält laut gaschromatographischer Analyse 97,7 % Isophorondiamin und 0,3 % 1,3,3-Trimethyl-6-azabicyclo[3.2.1]octan, entsprechend einer Diamin-Ausbeute von 98,7.

### Beispiel 5

Ein vertikaler Rohrreaktor (Durchmesser: 16 mm, Füllhöhe: 100 cm, ölbeheizter Doppelmantel) wurde mit 354 g (200 ml) eines basischen Cobalt-Vollkontaktes (CoO mit 5 % Mn₂O₃ und 1,4 % Na₂O) in Form von 1 bis 1,5 mm Split gefüllt. Zur Reduktion des Katalysators wurde bei 100 bar unter gleichzeitigem Durchleiten von 150 Nl/h Wasserstoff die Temperatur innerhalb von 23 h schrittweise von 100 auf 330°C erhöht und dann 30 h bei 330°C gehalten.

Durch einen vor den Hydrierreaktor geschalteten Rohrreaktor (Durchmesser: 16 mm, Füllhöhe: 20 cm, ölbeheizter Doppelmantel), der mit 25,4 g (40 ml) TiO₂ (Anatas) in Form von 1,5 mm-Strängen gefüllt war, werden bei einem Druck von 250 bar und einer Temperatur von 80°C stündlich von unten nach oben 40,0 g geschmolzenes Isophoronnitril (Reinheit 99,0 %) und 102 g flüssiges Ammoniak gepumpt. Anschließend werden stündlich 100 Nl/h Wasserstoff zugefahren und der Austrag aus dem vorgeschalteten Iminierungsreaktor bei einem Druck von 250 bar und einer Temperatur von 110°C von unten nach oben durch den Hydrierreaktor gefahren. Nach Entspannen auf Normaldruck wird der Ammoniak abdestilliert. Der Hydrieraustrag enthält laut gaschromatographischer Analyse 97,5 % Isophorondiamin. Der Austrag aus 73 h wird gesammelt und durch fraktionierende Destillation über eine 30 cm Füllkörperkolonne (3 mm Glasringe) aufgetrennt. Man erhält 2864 g Isophorondiamin, entsprechend einer Ausbeute von 96,2 % d.Th..

## Patentansprüche

1. Verfahren zur Herstellung von 3-Aminomethyl-3,5,5-trimethyl-cyclohexylamin aus 3-Cyano-3,5,5-trimethyl-cyclohexanon, dadurch gekennzeichnet, daß man in zwei räumlich voneinander getrennten Reaktionsräumen
a) das 3-Cyano-3,5,5-trimethyl-cyclohexanon in einem ersten Reaktionsraum mit überschüssigem Ammoniak an aciden Metalloxidkatalysatoren bei Temperaturen von 20 bis 150°C und Drücken von 15 bis 500 bar umsetzt und
b) die entstandenen Reaktionsprodukte in einem zweiten Reaktionsraum mit Wasserstoff in Gegenwart von überschüssigem Ammoniak an cobalt-, nickel-, ruthenium- und/oder anderen edelmetallhaltigen Katalysatoren gegebenenfalls mit basischen Komponenten oder auf neutralen oder basichen Trägern bei Temperaturen von 60 bis 150°C und Drücken von 50 bis 300 bar hydriert.

2. Verfahren nach Anspruch 1, dadurch gekennzeichnet, daß die cobalt-, nickel-, ruthenium- und/oder anderen edelmetallhaltigen Katalysatoren als basische Komponenten Oxide oder Hydroxide von Alkali- oder Erdalkalimetallen enthalten.

## Claims

1. A process for the preparation of 3-aminomethyl-3,5,5-trimethylcyclohexylamine from 3-cyano-3,5,5-trimethylcyclohexanone, wherein, in two spatially separate reaction spaces,
a) the 3-cyano-3,5,5-trimethylcyclohexanone is reacted in a first reaction space with excess ammonia on acidic metal-oxide catalysts at from 20 to 150°C and at from 15 to 500 bar, and
b) the resultant reaction products are hydrogenated, in a second reaction space, with hydrogen in the presence of excess ammonia on cobalt-, nickel-, ruthenium- and/or other noble metal-containing catalysts, with or without basic components or on neutral or basic supports, at from 60 to 150°C and at from 50 to 300 bar.

2. A process as claimed in claim 1, wherein the cobalt-, nickel-, ruthenium- and/or other noble metal-containing catalysts contain, as basic components, oxides or hydroxides of alkali or alkaline earth metals.

## Revendications

1. Procédé pour préparer la 3-aminométhyl-3,5,5-triméthylcyclohexylamine à partir de 3-cyano-3,5,5-triméthyl-cyclohexanone, caractérisé en ce que, séparément dans deux chambres de réaction distinctes ;
a) on fait réagir à des températures de 20 à 150°C sous des pressions de 15 à 500 bars la 3-cyano-3,5,5-triméthylcyclohexanone dans une première chambre de réaction, avec de l'ammoniac en excès, sur des catalyseurs acides à base d'oxyde métallique ; et
b) dans une deuxième chambre de réaction, à des températures de 60 à 150°C et sous des pressions de 50 à 300 bars, on hydrogène avec de l'hydrogène les produits de réaction ainsi obtenus, en présence d'ammoniac en excès, sur des catalyseurs contenant du cobalt, du nickel, du ruthénium et/ou d'autres métaux précieux, éventuellement avec des composants basiques ou encore sur des supports neutres ou basiques.

2. Procédé selon la revendication 1, caractérisé en ce que les catalyseurs contenant du cobalt, du nickel, du ruthénium et/ou d'autres métaux précieux, contiennent comme composants basiques des oxydes ou hydroxydes de métaux alcalins ou alcalinoterreux.
